(19)

**Europäisches Patentamt**
**European Patent Office**
**Office européen des brevets**

(11) **EP 4 342 373 B1**

(12) **EUROPEAN PATENT SPECIFICATION**

(45) Date of publication and mention
of the grant of the patent:
**06.05.2026 Bulletin 2026/19**

(21) Application number: **22836837.9**

(22) Date of filing: **04.07.2022**

(51) International Patent Classification (IPC):
*A61B 5/055* (2006.01)　　*A61B 5/00* (2006.01)
*A61N 1/36* (2006.01)　　*A61N 1/372* (2006.01)
*A61N 5/06* (2006.01)　　*G06T 7/00* (2017.01)

(52) Cooperative Patent Classification (CPC):
**A61N 1/3605; A61B 5/055; A61B 5/4064;**
**A61B 5/4836; A61N 1/37282;** A61B 2576/026;
A61N 5/0622; G06T 7/0012

(86) International application number:
**PCT/CN2022/103558**

(87) International publication number:
**WO 2023/280086 (12.01.2023 Gazette 2023/02)**

(54) **TARGET DETERMINATION METHOD AND APPARATUS, ELECTRONIC DEVICE, STORAGE MEDIUM, AND NEUROMODULATION DEVICE**

ZIELBESTIMMUNGSVERFAHREN UND -VORRICHTUNG, ELEKTRONISCHE VORRICHTUNG, SPEICHERMEDIUM UND NEUROMODULATIONSVORRICHTUNG

PROCÉDÉ ET APPAREIL DE DÉTERMINATION DE CIBLE, DISPOSITIF ÉLECTRONIQUE, SUPPORT DE STOCKAGE ET DISPOSITIF DE NEUROMODULATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.07.2021 CN 202110757474**

(43) Date of publication of application:
**27.03.2024 Bulletin 2024/13**

(73) Proprietor: **Beijing Galaxy Circumference Technologies Co., Ltd.**
**Beijing 102206 (CN)**

(72) Inventors:
• **WEI, Kecheng**
**Beijing 102206 (CN)**

• **WANG, Yezhe**
**Beijing 102206 (CN)**
• **ZHANG, Wei**
**Beijing 102206 (CN)**
• **ZHANG, Qiong**
**Beijing 102206 (CN)**

(74) Representative: **Metida**
**Gyneju str. 16**
**01109 Vilnius (LT)**

(56) References cited:
WO-A2-2009/044270　　CN-A- 108 355 250
CN-A- 109 480 841　　CN-A- 111 407 276
CN-A- 112 546 446　　CN-A- 113 367 679
US-A1- 2015 119 689　　US-A1- 2016 260 224
US-A1- 2020 214 581　　US-A1- 2021 170 180

EP 4 342 373 B1

Description

## TECHNICAL FIELD

. The present disclosure relates to the field of computer technology, in particular, to a method and a device for target identification, an electronic apparatus, a storage medium and a neuromodulation apparatus.

## BRIEF DESCRIPTION OF THE RELATED ART

. US patent publication US 2015/119689A1 discloses Systems and methods for treatment of neurological conditions using aTBS in accordance with embodiments of the invention are illustrated. One embodiment includes a method for treating neurological conditions, including generating a personalized accelerated theta burst stimulation (aTBS) target representing a location in a patient's brain related to a neurological condition, placing a TMS device relative to the patient's brain such that the focus of the magnetic field is over the aTBS target, applying TMS to the TBS target according to an aTBS protocol to the brain using the TMS device to alleviate the neurological condition.

. US patent publication US 2021/170180A1 discloses A method of performing personalized neuromodulation on a subject is provided. The method includes acquiring functional magnetic resonance imaging (fMRI) data of a brain of the subject. The method also includes calculating functional connectivity of the brain between a voxel in a subcortical region of the brain and a voxel in a cortical region of the brain, based on the fMRI data. The method also includes identifying a target location in the brain to be targeted by neuromodulation based on the calculated functional connectivity.

. A variety of neurological and psychiatric disorders often do not have a clear pathogenic focus, but only manifest as abnormal neurological function. The use of neuromodulation methods such as electricity, magnetism, light and ultrasound to directly or indirectly adjust the abnormal functional network is an important approach to improve the symptoms of patients. The challenge lies in determining neuromodulation targets in the human brain. Studies have shown that for most neurological and psychiatric disorders, it is typically impossible to achieve the desirable modulation and therapeutic effect since the etiology of the disease and the location of the disorder focus cannot be directly obtained from the structural images. Therefore, there is a clinical need for an objective, accurate, and quantifiable auxiliary method to help physicians screen individualized neuromodulation targets. Existing methods for identifying neuromodulation targets do not meet this need.

## SUMMARY OF THE DISCLOSURE

. The present disclosure proposes out a method and a device for target identification, an electronic apparatus, a storage medium and a neuromodulation apparatus for screening individualized neuromodulation targets.

. In a first aspect, the present disclosure provides a method of target identification, the method comprising: acquiring scanning data of a subject, wherein the scanning data comprise the data acquired from magnetic resonance imaging of the brain of the subject; determining at least two regions of interest(ROI) of the subject based on the scanning data; determining at least one abnormal region of interest(ROI) in the at least two regions of interest in accordance with a predetermined anomaly detection rule; determining a target based on the at least one abnormal region of interest.

. In some optional embodiments, determining the at least two regions of interest of the subject based on the scanning data comprises:

determining connectivity between each two voxels in the scanning data to form a brain connectivity matrix corresponding to the scanning data;

forming the at least two regions of interest based on a brain region template of a standard brain and the brain connectivity matrix.

. In some optional embodiments, determining the at least two regions of interest of the subject based on the scanning data comprises:

determining the connectivity between each two voxels in the scanning data;

dividing the scanning data corresponding to the anatomical structure of the brain of the subject into a plurality of big regions, dissecting each of the plurality of big regions into a plurality of brain regions, wherein each of the plurality of brain regions comprises at least one voxel;

fusing brain regions of the plurality of brain regions having a voxel connectivity between the brain regions above a predetermined brain region voxel connectivity threshold to form the at least two regions of interest.

. In some optional embodiments, determining the at least two regions of interest of the subject based on the scanning data, comprises:

determining the at least two regions of interest of the subject based on a volume standard brain template according to the scanning data.

. In some optional embodiments, determining the at least two regions of interest of the subject based on the scanning data, comprises:

determining the at least two regions of interest of the subject based on a cortical standard brain template according to the scanning data.

. In some optional embodiment, determining at least one abnormal region of interest in the at least two regions of interest in accordance with a predetermined anomaly detection rule, comprises:

acquiring group brain magnetic resonance data;
determining a group brain connectivity matrix based on the group brain magnetic resonance data;
determining connectivity between each two voxels in the scanning data to form a brain connectivity matrix corresponding to the scanning data;
determining the at least one abnormal region of interest in accordance with the group brain connectivity matrix and the brain connectivity matrix.

. According to the claimed invention, determining a target based on the at least one abnormal region of interest comprises:

determining whether the at least one abnormal region of interest is located in a modulation brain region or not;
if the at least one abnormal region of interest is located in the modulation brain region, determining a center of the at least one abnormal region of interest as the target, or, determining a region with the center of the at least one abnormal region of interest as a spherical center and with a predetermined target radius as a first target region of interest(TROI), determining the target based on a position of the first target region of interest;
if the at least one abnormal region of interest is not located in the modulation brain region, determining the connectivity of the at least one abnormal region of interest with other regions of interest in the at least two regions of interest, and the region of interest among the other regions of interest having the connectivity with the at least one abnormal region of interest that exceeds a predetermined connectivity threshold and which is located in the modulation region as a second target candidate;
determining a center of the second target candidate as the target, or determining a region with the center of the second target candidate as a spherical center and with the predetermined target radius as the second target region of interest, determining the target based on a position of the second target region of interest.

. In some optional embodiment, determining the target based on the at least one abnormal region of interest comprises:

determining a brain structure subdivision in which the target is located based on a disease type of the subject;
determining an intersection of the at least one abnormal region of interest or the region of interest whose connectivity with the abnormal region of interest satisfies a predetermined connectivity threshold condition with the brain structure subdivision as a target candidate;
determining a center of the target candidate as the target, or, determining a region with the center of the target candidate as a spherical center and with a predetermined target radius as the target region of interest, and determining the target based on a position of the target region of interest.

. In some optional embodiments, the functional magnetic resonance imaging comprises: structural magnetic resonance imaging, and/or, task-based functional magnetic resonance imaging, and/or, resting state functional magnetic resonance imaging.

. In a second aspect, the present disclosure provides a device for target identification comprising: a data acquisition unit, configured to acquire scanning data of the subject, wherein the scanning data comprise data acquired from magnetic resonance imaging of the brain of the subject; a processing unit, configured to determine at least two regions of interest of the subject based on the scanning data; an anomaly detection unit, configured to determine at least one abnormal region of interest in the at least two regions of interest based on a predetermined anomaly detection rule; a target identification unit, configured to determine a target based on the at least one anomaly region of interest.

. In some optional embodiments, the processing unit is further configured to:

determine connectivity between each two voxels in the scanning data to form a brain connectivity matrix corresponding to the scanning data;

form the at least two regions of interest based on a brain region template of a standard brain and the brain connectivity matrix.

. In some optional embodiments, the processing unit is further configured to:

determine the connectivity between each two voxels in the scanning data;
divide the scanning data corresponding to the anatomical structure of the brain of the subject into a plurality of big regions, dissect each of the plurality of big regions into a plurality of brain regions, wherein each of the plurality of brain regions comprises at least one voxel;
fuse brain regions of the plurality of brain regions having a voxel connectivity between the brain regions above a predetermined brain region voxel connectivity threshold to form the at least two regions of interest.

. In some optional embodiments, the processing unit is further configured to:
determine the at least two regions of interest of the subject based on a volume standard brain template according to the scanning data.
. In some optional embodiments, the processing unit is further configured to:
determine the at least two regions of interest of the subject based on a cortical standard brain template according to the scanning data.
. In some optional embodiments, the anomaly detection unit is further configured to:

acquire group brain magnetic resonance data;
determine a group brain connectivity matrix based on the group brain magnetic resonance data;
determine connectivity between each two voxels in the scanning data to form a brain connectivity matrix of the subject corresponding to the scanning data;
determine the at least one abnormal region of interest in accordance with the group brain connectivity matrix and the brain connectivity matrix of the subject.

. In some optional embodiments, determining a target based on the at least one abnormal region of interest comprises:

determining whether the at least one abnormal region of interest is located in a modulation brain region or not;
if the at least one abnormal region of interest is located in the modulation brain region, determining a center of the at least one abnormal region of interest as the target, or, determining a region with the center of the at least one abnormal region of interest as a spherical center and with a predetermined target radius as a first target region of interest, determining the target based on a position of the first target region of interest;
if the at least one abnormal region of interest is not located in the modulation brain region, determining the connectivity of the at least one abnormal region of interest with other regions of interest in the at least two regions of interest, and the region of interest among the other regions of interest having the connectivity with the at least one abnormal region of interest that exceeds a predetermined connectivity threshold and which is located in the modulation region as a second target candidate;
determining a center of the second target candidate as the target, or determining a region with the center of the second target candidate as a spherical center and with the predetermined target radius as the second target region of interest, determining the target based on a position of the second target region of interest.

. In some optional embodiments, determining the target based on the at least one abnormal region of interest comprises:

determining a brain structure subdivision in which the target is located based on a disease type of the subject;
determining an intersection of the at least one abnormal region of interest or the region of interest whose connectivity with the abnormal region of interest satisfies a predetermined connectivity threshold condition with the brain structure subdivision as a target candidate;
determining a center of the target candidate as the target, or, determining a region with the center of the target candidate as a spherical center and with a predetermined target radius as the target region of interest, and determining the target based on a position of the target region of interest.

. In some optional embodiments, the magnetic resonance imaging comprises: structural magnetic resonance imaging, and/or, task-based functional magnetic resonance imaging, and/or, resting state functional magnetic resonance imaging.

. In a third aspect, the present disclosure provides an electronic apparatus, comprising:

at least one processor; and a storage device having at least one program stored thereon,
wherein the at least one program, when executed by the at least one processor, causes the at least one processor to execute the method as described in any implementation of the first aspect.

. In a fourth aspect, the present disclosure provides a computer readable storage medium having a computer program stored thereon, wherein the computer program, when executed by at least one processor, executes a method as described in any implementation of the first aspect.

. In a fifth aspect, the present disclosure provides a neuromodulation apparatus, configured to make neuromodulation on the target of the subject in accordance with the preset neuromodulation solution; wherein the target is determined by the method according to any implementation of the first aspect.

. In some optional embodiments, said preset neuromodulation solution comprises at least one of the following:

deep brain electrical stimulation;
transcranial electrical stimulation;
electroconvulsive therapy;
electrical stimulation based on cortical brain electrodes;
transcranial magnetic stimulation;
focused ultrasound neuromodulation;
magnetic resonance guided high-intensity focused ultrasound therapy neuromodulation; and
photobiomodulation therapy.

. In order to achieve the determination of neuromodulation targets, current commonly used technical means includes:

1. Based on group-level task-based functional magnetic resonance imaging (fMRI) to determine neuromodulation targets; the shortcomings of this approach include: task-based fMRI signal-to-noise ratio is low, reproduce-ability is not high, and subjects are required to have a certain level of cognitive level; the results of the functional regions of the task-based fMRI are greatly affected by the design of the task; and it is difficult to determine the baseline level of the functional regions.

2. With the aid of clinical experience based on the anatomical structure of the brain, the approximate location of the body projection of a specific functional region on the surface of the patient's scalp is found to determine the neuromodulation target, such as the repetitive transcranial magnetic stimulation (rTMS) DLPFC location method (known as the left dorsal lateral prefrontal cortex) for the treatment of refractory depression approved by the U.S. Food and Drug Administration (FDA). DLPFC location method is often referred to as the "5-cm" location method; the shortcomings of this approach include ignoring individual anatomical differences and having low location accuracy, resulting in imprecise location of neuromodulation targets; ignoring individual functional network differences, and locating targets in other functional regions of the brain.

3. According to the electrode cap to determine the neuromodulation target, such as the international 10-20 electrode cap location method; the shortcomings of the method include: ignoring the individual anatomical structure differences and low location accuracy, resulting in imprecise location of neuromodulation target; ignoring the differences in individual functional networks.

4. Determining neuromodulation targets based on anatomical structures or ROIs defined by group-averaged fMRI studies; the shortcomings of this approach include: a variety of neurological and psychiatric disorders often do not have clear pathogenic focus, but only show abnormalities in nervous system function, and purely anatomical structures are unable to reflect the characteristics of the disorders; the etiology of neurological and psychiatric disorders is complex, and coupled with individual differences, the therapeutic solutions based on group-averaged fMRI have low efficacy rates.

5. According to the metabolism of the tissue structure reflected in the PET scanning data to determine the neuromodulation target; the shortcomings of this approach include: expensive PET scanning, thus increasing the burden of medical care; there is a certain amount of radiation in the scanning process; PET scanning is applicable to a limited number of neurological and psychiatric disorders; the signal-to-noise ratio of the image is low, and the boundaries of the anatomical structure are not clear enough to affect the accuracy of determining the target, so that the effectiveness of the clinical treatment is low.

. The present disclosure provides a method and a device for target identification, an electronic apparatus, a storage medium, and a neuromodulation apparatus, wherein scanning data of the subject is acquired, wherein the scanning data comprise data acquired from performing the magnetic resonance imaging of the brain of the subject, determining the at least two regions of interest of the subject based on the scanning data; determining at least one abnormal region of interest in the at least two regions of interest in accordance with a predetermined anomaly detection rule; determining a target based on the at least one abnormal region of interest. Embodiments of the present disclosure utilize functional magnetic resonance imaging to provide scanning data of the brain of the subject and determine the brain region of the subject, which can effectively solve the problem of inaccurate neuromodulation target caused by traditional methods that do not take into account the structural or functional differences of an individual, on the basis of full consideration of individual difference, and realize the location of individualized neuromodulation target of the subject. The individualized abnormality detection method can efficiently detect brain regions with abnormal brain connectivity patterns compared with normal subjects by combining brain structural and functional information, and can effectively solve the problem of inaccurate neuromodulation targets caused by traditional methods that do not consider individual structural or functional differences.

## BRIEF DESCRIPTION OF THE DRAWINGS

. Various embodiments discussed herein will be generally shown in drawings by way of example, without limitation.

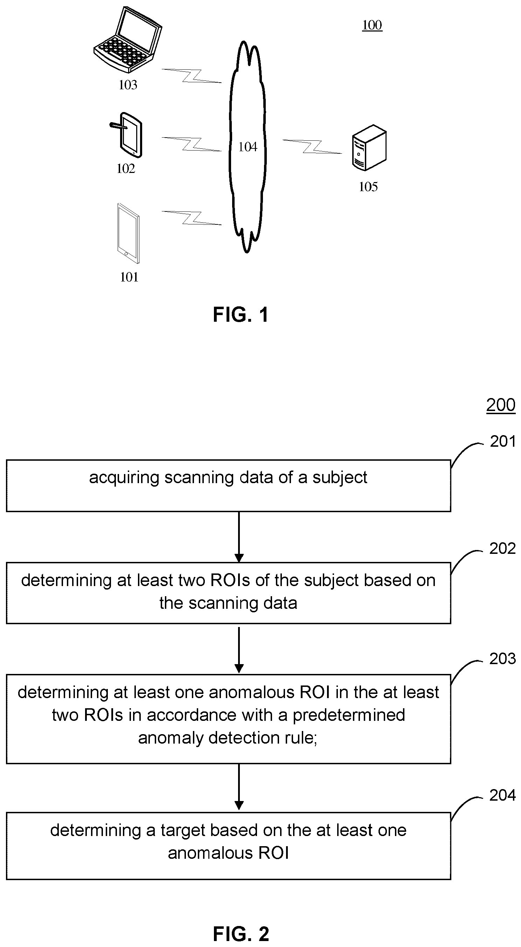

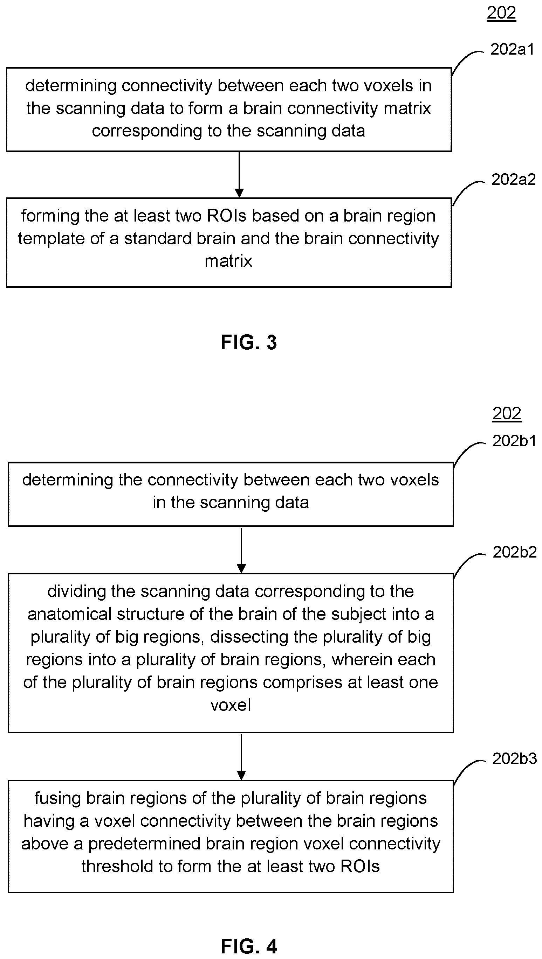

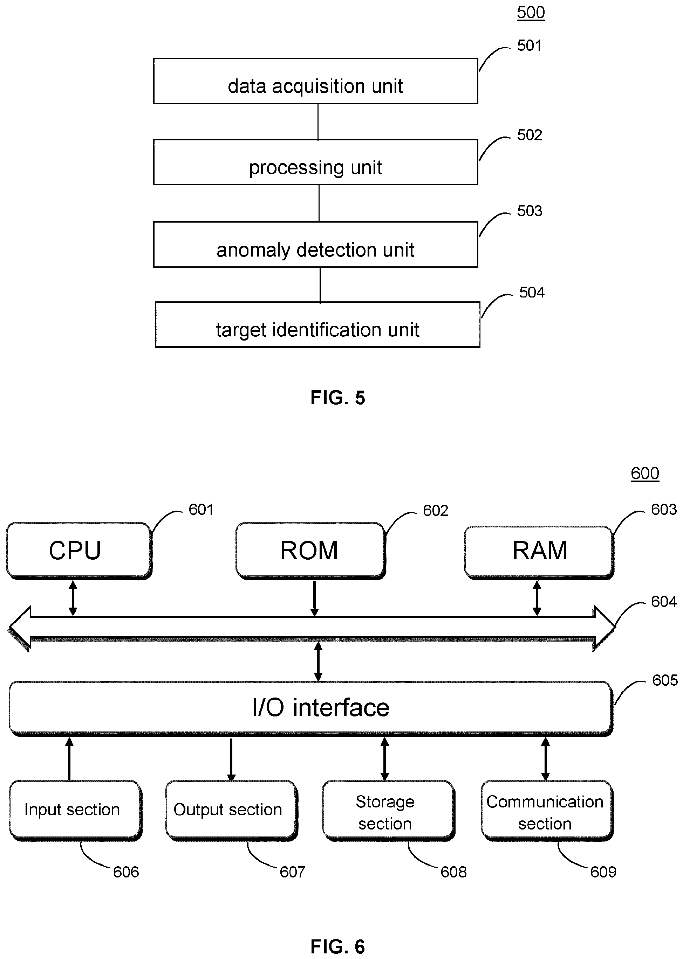

. FIG. 1 is an exemplary system architecture diagram in which an embodiment of the present disclosure may be applied;
. FIG. 2 is a flow schematic diagram of one embodiment of the method for target identification in accordance with the present disclosure.
. FIG. 3 is a schematic diagram of a detailed flow of an embodiment of step 202 of the the method for target identification shown in FIG. 2;
. FIG. 4 is a schematic diagram of a detailed flow of another embodiment of step 202 of the method for target identification shown in FIG. 2;
. FIG.5 is a schematic structural diagram of an embodiment of the device for target identification according to the present disclosure;
. FIG. 6 is a schematic diagram of a structure of a computer system suitable for implementing a terminal equipment or server of the present disclosure.

## DETAILED DESCRIPTION OF THE PREFERRED EMBODIMENT

. In order that the characteristics and technical contents of the embodiments of the present disclosure can be understood in detail, a more detailed description on how to implement the embodiments of the present disclosure will be given by reference to the accompanying drawings. These drawings are only intended to illustrate, but are not intended to limit the embodiments of the present disclosure.

. In the description of embodiments of the present disclosure, it is to be noted that, unless otherwise specified and limited, the term "connection" is to be understood in a broad sense, for example, it may be an electrical connection, it may be a connection between two elements, it may be a direct connection, it may be an indirect connection through an intermediary medium, and a person of ordinarily skilled in the art may be able to understand the specific meanings of the aforesaid terms in accordance with the specific circumstances.

. It should be noted that the terms "first\second\third" related to the embodiments of the present disclosure are only used to distinguish similar objects, but do not mean a specific ordering for the objects. And it should be understood that "first \ second\ third" may be interchanged in the specific order or sequence when allowed. It should be noted that the objects defined by "first\ second \third" may be interchanged under appropriate circumstances such that embodiments of the present disclosure described herein may be implemented in orders other than those illustrated or described herein.

. FIG. 1 illustrates an exemplary system architecture 100 to which embodiments of a method or a device for target identification of the present disclosure can be applied.

. As shown in FIG. 1, the system architecture 100 can include terminal devices 101, 102, 103, a network 104, and a server 105. The network 104 is a medium used to provide communication links between the server 105 and each of the terminal devices 101, 102, 103. The network 104 may include various connection types, such as wired communication links, wireless communication links, or fiber optic cables, and so on.

. Users may use terminal devices 101, 102, 103 to interact with the server 105 over the network 104 to receive or transmit messages or the like. Various communication client applications, such as a magnetic resonance imaging control application, a functional magnetic resonance imaging control application, a web browser application, a shopping application, a search application, an instant messaging tool, a mailbox client, a social platform software, and the like, may be installed on the terminal devices 101, 102, and 103.

. The terminal devices 101, 102, 103 can be implemented by hardware or software. When the terminal devices 101, 102, 103 are implemented by hardware, they may be various electronic apparatuses with display screens, including but not limited to smart phones, tablet computers, laptop portable computers, desktop computers, and the like. When the terminal devices 101, 102, 103 are implemented by software, they can be installed in the above-listed electronic apparatuses for determining brain regions of the subject. It may be implemented as a plurality of software or software modules (e.g. processing for providing the brain maps) or as a single software or software module. It is not particularly limited herein.

. The server 105 can be a server that provides various services, such as a background data processing server that processes scanning data transmitted by the terminal devices 101, 102, 103. The background data processing server can determine the brain regions of the subject based on the scanning data and the voxel corresponding to each brain region, and send them back to the terminal devices.

. It should be noted that the server 105 can be implemented by hardware or software. When the server 105 is implemented by hardware, it may be implemented as a distributed server cluster composed of a plurality of servers, or may be implemented as a single server. When the server 105 is implemented by software, it may be implemented as a plurality of software or a software module (e.g., to provide distributed services), or as single software or a software module. It is not particularly limited herein.

. It should be noted that the method for determination target as provided by the present disclosure is generally performed by the server 105, and accordingly, the device for determination target is generally disposed in the server 105.

. It should be noted that, in some cases, the method for determination target provided by the present disclosure may be executed by the server 105, by the terminal devices 101, 102, and 103, or both the server 105 and the terminal devices 101, 102, and 103. Accordingly, the device for determination target may be disposed in the server 105, or may be disposed in the terminal devices 101, 102, and 103, or may be disposed partially in the server 105 or partially in the terminal devices 101, 102, and 103. And accordingly the system architecture 100 can include only the server 105, or only the terminal devices 101, 102, 103, or can include the terminal devices 101, 102, 103, the network 104 and the server 105. It is not particularly limited in the present disclosure.

. It should be understood that the numbers of the terminal devices, the network, and the server in FIG. 1 is merely illustrative. There may be any number of terminal devices, networks, and servers, as desired for an implementation.

. Continuing to refer to FIG. 2, it illustrates a flow 200 of an embodiment of the method for target identification according to the present disclosure, comprising the following steps:

. Step 201, the scanning data of the subject is acquired.

. In some embodiments of the present disclosure, the scanning data comprise data acquired from magnetic resonance imaging of brain of the subject.

. The scanning data comprise a Blood Oxygen Level Dependency (BOLD) signal sequence corresponding to each of the predetermined number of voxels.

. In present embodiment, an executing subject (e.g., the server shown in FIG. 1) of the method for target identification may first locally or remotely acquire the scanning data of the subject from other electronic apparatus(e.g., the terminal device shown in FIG. 1) connected to the network of the executing subject.

. Voxels, also known as stereo pixels (voxel), are short for volume pixel. A voxel is conceptually similar to a pixel, the smallest unit of two-dimensional space, which is used in imaging data of two-dimensional computer images . A voxel is the smallest unit of digital data in a three-dimensional spatial segmentation, and is used in the fields of three-dimensional imaging, scientific data, and medical imaging.

. The BOLD signal sequence corresponding to a voxel means that the subject is scanned by magnetic resonance, and then a BOLD signal is obtained for each voxel at preset time units, and finally the BOLD signals for a period of time are obtained, and these BOLD signals are arranged in the order of the acquisition time, i.e., the BOLD signal sequence corresponding to each voxel is obtained, where the number of the BOLD signals included in the sequence is an integer quotient of the time duration corresponding to the target task divided by a preset time unit. For example, if the time duration corresponding to the scanning is 300 seconds, and the preset time unit is 2 seconds, then there are 150 BOLD values in the BOLD signal sequence corresponding to each voxel, and it can also be considered that there are 150 frames of data in the BOLD signal sequence corresponding to each voxel, or it can also be considered that the BOLD signal sequence corresponding to each voxel is a vector with a dimensionality of 150, or it can also be considered that the BOLD signal sequence corresponding to each voxel is seemed as a 1 x 150 order matrix, to which the present disclosure is not specifically limited.

. It is understood that the specific number of voxels included in the scanning data may be determined according to the scan accuracy of the functional magnetic resonance imaging or the magnetic resonance imaging, and may also be determined according to the accuracy of the imaging device, and the predetermined number herein is not a limit for the specific number of voxels, and in the current practical application, the number of voxels in the scanning data of the human brain is measured in terms of ten thousand or one hundred thousand, and with the advancement of the scanning technology, the number of voxels included in the scanning data of the human brain is capable of being further improved.

. In the present disclosure, the performing subject as described above may acquire the scanning data of the subject locally or remotely from other electronic apparatus (e.g., the terminal device shown in FIG. 1) connected to the network of the performing subject.

. In embodiments of the present disclosure, the magnetic resonance imaging may include: structural magnetic resonance imaging, and/or, task-based functional magnetic resonance imaging, and/or, resting state functional magnetic resonance imaging.

. The data acquired from functional magnetic resonance imaging contain information of time sequences, which is equivalent to a four-dimensional image. For example, if a functional magnetic resonance imaging image is acquired with a 3-dimensional image matrix (Length $\times$ Width $\times$ Height, L $\times$ M $\times$ N), and one frame is acquired every 2 seconds, then 150 frames of data can be acquired in 6 minutes, forming a functional magnetic resonance imaging data signal of voxel L $\times$ M $\times$ N $\times$ 150.

. The data acquired from structural magnetic resonance imaging is a high-resolution three-dimensional gray-scale image of anatomical structures, such as T1w (T1-weighted imaging - salient tissue T1 relaxation (longitudinal relaxation) difference) and its associated images, T2w (T2-weighted imaging ---- salient tissue T2 relaxation (transverse relaxation) difference) and its associated images, Fluid Attenuated Inversion Recovery Sequence (FLAIR) and its associated images; Structural magnetic resonance imaging may also include magnetic resonance diffusion imaging, such as diffusion-weighted imaging (DWI) and its associated images, diffusion tensor imaging (DTI) and its associated images.

. DTI is a magnetic resonance technique used to study the diffusion anisotropy of anatomical nerve bundles of the central nervous system and to show the fiber anatomy of the white matter, in order to probe the tissue micro structure by the diffusion anisotropy of water molecules in the tissue. The anisotropy of cerebral white matter is due to parallel-traveling myelinated axonal fibers, and the dispersion of cerebral white matter is greatest in the direction of the parallel nerve fibers, i.e., the fractional anisotropy (FA) of the dispersion is greatest, and can be approximated to be 1 (which in reality can be a fraction greater than 0.9 and converging to 1). This property is reflected in the spatial directionality of brain white matter using color markers, i.e., the direction of fastest diffusion indicates the direction of fiber travel. Fiber bundle imaging by DTI can obtain the brain connectivity matrix that reflects the structure of the brain.

. In the embodiment of the present disclosure, the functional magnetic resonance imaging can include task-based functional magnetic resonance imaging and/or resting state functional magnetic resonance imaging.

. It will be appreciated that resting state functional magnetic resonance imaging is magnetic resonance imaging acquired from performing a magnetic resonance scan of the subject's brain while the subject is not performing any task during the scan. Accordingly, task-based functional magnetic resonance imaging is magnetic resonance imaging acquired from performing a magnetic resonance scan of the subject's brain while the subject is performing a target task.

. After acquiring the structural magnetic resonance scanning data of the brain of the subject, various implementations can be used to determine a structural map of the brain of the subject based on the structural magnetic resonance scanning data of the brain of the subject, i.e., to obtain specific regions of the brain of the subject are meant to what structural components. For example, this can be implemented using existing software (such as the magnetic resonance data processing software Free Cortical Reconstruction (FreeSurfer)) for processing 3D brain scanning data. As another example, it is also possible to train in advance a deep learning model based on a large amount of brain structure image scan sample data and the annotation of the corresponding brain structural components, and then input the subject's brain structure magnetic resonance scanning data into the trained deep learning model and obtain the corresponding brain structure map.

. In some optional embodiments, the above-described executing subject pre-processes the scanning data of the subject after acquiring the scanning data of the subject.

. Without specifically limiting a method step of the pre-processing in the present disclosure, exemplarily, the pre-processing may comprise:

. pre-processing the functional magnetic resonance imaging images, e.g.,

(1) temporal layer correction, head movement correction, temporal signal filtering, noise component regression, spatial smoothing, and so on;
(2) functional magnetic resonance imaging image alignment to the structural image (if there is a structural image);
(3) functional magnetic resonance imaging signal projection to structural images (if the structural images are available), including reconstructed individual cortical images or structural images averaged over the relevant group.

. pre-processing the structural MRI images (if structural images are available), e.g., skull removal, field strength correction, segmentation of individual anatomical structures, cerebral cortex reconstruction, etc.

. Step 202, at least two ROIs of the subject are determined based on the scanning data.

. For the above step 202, the present disclosure provides a variety of optional implementations.

. FIG.3 is a partially detailed schematic diagram of one embodiment of step 202 of the method for target identification shown in FIG.2. In some optional embodiments of the application, as shown in FIG.3, the above step 202 may specifically include:

. Step 202a1, determining connectivity between each two voxels in the scanning data to form a brain connectivity matrix corresponding to the scanning data.

. In the present disclosure, the connectivity of each voxel to the ROI may include a mean of the connectivity of the voxel to each voxel in the ROI; the connectivity between two ROIs may include a mean of the connectivity of the voxel in each of the two ROIs to each voxel in the other ROI; the connectivity of the voxel to the brain region may include a mean of the connectivity of the voxel to each voxel in the brain region; and the connectivity between two brain regions may include a mean of the connectivity of the voxel in each of the two brain regions to each voxel in the other brain region.

. A connectivity characterizes the connectivity of the brain connections, which may also be expressed as the correlation. Here, the brain connectivity may include functional connectivity and structural connectivity. Functional connectivity may be obtained by calculating a Pearson correlation coefficient based on the BOLD time sequence corresponding to voxels within ROIs; the structural connectivity includes, for example, structural connectivity between ROIs obtained based on fiber bundle imaging.

. Exemplarily, assuming that the number of voxels in the scanning data is 100,000, the BOLD signal sequence corresponding to each voxel includes T BOLD values, and T is the number of samples in the time dimension corresponding to the scan time, the brain connectivity matrix corresponding to the scanning data is a 100,000x100,000-order matrix, the brain connectivity matrix is capable of characterizing the connectivity between each two voxels in the scanning data; wherein the connectivity between the two voxels can be calculated by a Pearson correlation coefficient based on the T BOLD values corresponding to the voxels.

. In the present disclosure, the correlation coefficient is a Pearson's correlation coefficient, which is a coefficient used to measure a linear degree between variables. The calculation formula thereof is as follows:

$$\rho_{X,Y} = \frac{\text{cov}(X, Y)}{\sigma_X \sigma_Y} = \frac{E((X - \mu_X)(Y - \mu_Y))}{\sigma_X \sigma_Y} = \frac{E(XY) - E(X)E(Y)}{\sqrt{E(X^2) - E^2(X)}\sqrt{E(Y^2) - E^2(Y)}}$$

. The formula is defined as: the Pearson's correlation coefficient $\rho_{(X,Y)}$ of two consecutive variables (X, Y) is equal to the covariance Cov (X, Y) between them divided by the product of their respective standard deviations ($\sigma_X$, $\sigma_Y$). Coefficients have always values between - 1.0 and 1.0. If the variables equal to or approximately equal to 0, it will be called as having no correlation. In contrast, if the variables equal to or approximately equal or approach to 1 or -1, it will be called as having strong correlation. Here, "approximately equal to" can be understood as the difference with the target value is within the error allowable range, for example, in the present disclosure, 0.01 can approximately equal to 0, or, 0.99 can approximately equal to 1. This is only by way of example, and the error allowable range of the "approximately equal to" can be determined for practical applications based on the accuracy required for the calculation.

. Step 202a2, at least two ROIs are formed based on the brain region template of the standard brain and the brain connectivity matrix.

. Exemplarily, a brain map comprising more than 2 brain regions may be created for the subject based on the brain region template of standard brain using pattern recognition or machine learning methods. Methods may include, but are not limited to, Independent Component Correlation Algorithm (ICA), Principal Component Analysis (PCA), various types of clustering methods, factor analysis, and Linear discriminant analysis (LDA), various matrix decomposition methods and so on. In the final brain functional network obtained functional subdivisions may include different positions of voxels for different subjects, but each voxel belongs to the specific ROI. That is, each ROI of the subject can be a collection of voxels composed of voxels with the same function in fMRI.

. In the embodiment of the present disclosure, the brain region can include brain function subdivisions and/or brain structural subdivisions.

. FIG.4 is a detail schematic diagram of yet another embodiment of step 202 of the method for target identification shown in FIG.2. In some optional embodiments, as shown in FIG.4, the above step 202 may specifically include:

. Step 202b1, determining the connectivity between each two voxels in the scanning data.

. Step 202b2, dividing an anatomical structure of the brain of the subject corresponding to the scanning data to a plurality of big regions, the big regions ( for example each big region) are dissected into a plurality of brain regions, wherein each brain region of the brain regions includes at least one voxel.

. Step 202b3, fusing the brain regions in the brain regions having the connectivity between the plurality of brain regions above the predetermined brain region voxel connection threshold, to form the at least two ROIs.

. Exemplarily, the brain of the subject is first divided into a plurality of big regions according to major anatomical structure boundaries; thereafter, each big region is dissected by using functional connectivity, and the connectivity of the voxels in each region is determined according to reliability(test-retest reliability). After obtaining the brain regions by dissecting each big region, these brain regions are fused based on the connectivity of voxels included therein, and the brain regions having voxels with high correlation are combined into a single ROI, exemplarily, finally at least two ROIs are determined in the whole brain.

. For example, an initial individual brain map can be divided into ten big regions by dividing each of the left and right cortex of the brain into five big regions: frontal lobe, parietal lobe, occipital lobe, temporal lobe, and pan-central sulcus regions. As another example, the brain can be divided into four regions according to advanced and lower cortex subdivisions, and left-right brains.

. In some optional embodiments, the above step 202 specifically includes:

selecting in advance or generating a group brain map as a brain map template, and projecting boundaries corresponding to at least two brain regions in the brain map template onto the brain scanning data of the subject. adjusting the boundaries of the at least two brain-brain regions according to the scanning data of the brain of the subject so that the adjusted boundaries of the brain-brain region are matched with at least the brain scanning data of the subject to form the at least two ROIs.

. Exemplarily, the group brain maps are first projected directly to the brain of the subject, followed by a recursive algorithm that progressively adjusts the boundaries of the brain regions projected by the group brain maps based on the anatomical brain map of the subject until the boundaries of the brain regions are stabilized. The recursive process will utilize the distribution of individual differences in the subjects' brain connectivity, as well as the subjects' own signal-to-noise ratios of the brain images, to determine the magnitude of the boundary adjustment of the brain-brain regions. Finally, the brain-brain regions are fused according to the voxel correlation, thus obtaining at least two ROIs.

. In some optional embodiments, the above step 202 may specifically comprise:
constructing a standard ROI library, and the ROI library includes a variety of test scenariorelated ROIs and their potential brain connectivity patterns. With the standard ROI library, the corresponding ROIs can be obtained by screening the test scenario types, and then at least two ROIs for the subject can be obtained on this basis.

. In some optional embodiments, the above step 202 may specifically comprise:
determining at least two ROIs for the subject based on the scanning data according to the volume standard brain structure template. It is feasible to extract the white matter and ventricular regions from the volume standard brain structure template, construct a binary Mask of the volume standard brain structure template, remove the white matter and ventricular regions from the Mask to obtain a Mask with no white matter and no ventricular regions, and resample the Mask with no white matter and no ventricular regions to obtain at least two ROIs. Alternatively, a binary Mask of the volume standard brain structure template is constructed and resampled to obtain at least two ROIs.

. In some optional embodiments, the above step 202 may specifically comprise:
determining at least two ROIs for the subject based on the scanning data according to the cortical standard brain structure templates. The at least two ROIs are generated by resampling the cortical standard brain structure templates. For example, at least two ROIs are generated for the high-resolution templates (e.g., fsaverage6(fs6)) based on the coarse-resolution cortical surface templates (e.g., fsaverage3(fs3), fsaverage4(fs4)). Specifically, the left and right brain vertices of the coarse-resolution template are assigned sequentially with numerical values (1,2,3...) respectively and then resampled into the fs6 template space (nearest-neighbor interpolation), and according to the order of assignment, counted all the vertex indexes in the fs6 surface corresponding to each value, which are at least two ROIs in the fs6 space,, the 13th vertex in the left brain fs4 surface template is assigned the value of 13, and after resampling into the fs6 template space, it can find 30 vertices with the value of 13, so the 13th ROI of the left brain fs6 surface template is composed of these 30 vertices. The at least two ROIs are generated based on all the vertices in the surface template, and for any surface template, each vertex will be used as one ROI, e.g. (the fsaverage6 left brain template consists of 40962 vertices, and 40962 ROIs can be generated accordingly).

. Step 203, determining at least one abnormal ROI in the at least two ROIs according to a predetermined anomaly detection rule.

. In some optional embodiments, the above step 203 may specifically comprise:
obtaining a group brain magnetic resonance data.

. Exemplarily, the number of group samples obtained may be 300 or more. This is not a specific limitation on the number of group samples, but only an example.

. The group brain magnetic resonance data obtained is pre-processed.

. The group brain connectivity matrix is determined from the group brain magnetic resonance data.

. Here, the calculation of the group brain connectivity matrix may include the following steps:
. Step S11, for data of each subject in the group brain magnetic resonance data, calculating average time sequences of all vertices/voxels in each ROI. Here, the ROIs may be obtained by the method of determining ROIs in embodiments of the present disclosure, or may be obtained by other existing methods of determining ROIs.

. Step S12, sequentially calculating the correlation coefficients (e.g., Pearson's correlation coefficient) between the ROIs and the ROI time sequences, and ultimately obtaining the brain connectivity matrix of each subject, which may include a functional connectivity matrix and a structural connectivity matrix. e.g., functional connectivity (FC) matrix, indi_fc (this matrix is a diagonal matrix, the values in the $i^{th}$ row and $j^{th}$ column of the matrix represent the correlation between the $i^{th}$ ROI and the $j^{th}$ ROI time sequence, i≤N, j≤N, N is the number of ROIs, and the size of indi_fc is N x N).

. The BOLD signal sequence corresponding to each voxel in the scanning data is obtained.

. Based on the BOLD signal sequence corresponding to each voxel, the connectivity between each two voxels in the scanning data is determined to form the brain connectivity matrix of the subject corresponding to the scanning data;

. At least one abnormal ROI is determined based on the group brain connectivity matrix and the brain connectivity matrix of the subject.

. In some embodiments, the step 203 may specifically include option 1 or option 2.

. Option 1: anomaly detection algorithm A, which may specifically include the following steps SA1 to SA3.

. Step SA1, generating a baseline (group brain connectivity matrix). Specifically, this may include the following sub-steps SA11 to SA14.

. Sub-step SA11, collecting data: collecting a large sample of fMRI data (generally, >=300 individuals).

. Sub-step SA12, pre-processing data: the data is pre-processed according to the pre-processing method in the embodiment of FIG. 2.

. Sub-step SA13, calculating the brain connectivity matrix.

. For the data of each subject in the large sample data, the average time sequences of all vertices/voxels in each ROI (generated by step SA1, assuming that there are N ROIs) is calculated.

. The correlation coefficients (e.g., Pearson's correlation coefficients) between ROIs and ROI time sequences are calculated sequentially, and finally the brain connectivity matrix of each sample is obtained, which is not limited to the functional connectivity matrix and the structural connectivity matrix, taking a functional connectivity (FC) matrix as an example, i.e., indi_fc (which is a diagonal matrix, the value in the $i^{th}$ row and the $j^{th}$ column of the matrix represent the correlation of the $i^{th}$ ROI with the $j^{th}$ ROI time sequence, i≤N, j≤N, N is the number of ROIs, and the size of indi_fc is N x N).

. Step SA14, generating a baseline.

. The indi_fc of all subjects is averaged to obtain the mean matrix, i.e. Big_mean_fc (the size of N x N).

. The correlation coefficients between each row of indi_fc and the corresponding row of Big_mean_fc are calculated sequentially, then obtaining a one-dimensional matrix (the size of N x 1), and finally the mean and standard deviation, i.e., Corr_mean (the size of N x 1) and Corr_std (the size of N x 1), are calculated for the one-dimensional matrix for all subjects.

. Step SA2: generating the subject FC (the brain connectivity matrix of the subject).

. Calculating the average time sequences of all vertices/voxels in each ROI (generated from step 1) by using the pre-processed data from the subject (being set as P).

. The correlation coefficients between the ROIs and the ROI time sequences (e.g., using Pearson's correlation coefficient) are calculated sequentially, to finally obtain the brain connectivity matrix of the subject P. The brain connectivity matrix may include a functional connectivity matrix and a structural connectivity matrix, and the functional connectivity (FC) matrix, for example, is set as p_fc (its size and meaning are the same as the indi_fc in step SA1).

. Calculating the correlation coefficients between each row of p_fc and the corresponding row of Big_mean_fc (generated by step SA1) sequentially, and obtaining an one-dimensional matrix of N x 1, e.g., p_corr.

. Step SA3, anomaly detection algorithm step.

. Performing the following operation on the $n^{th}$ (n ≤ N, N is the number of ROIs) ROI of p_corr.

$$p\_z(n) = \frac{|p\_corr(n) - Corr\_mean(n)|}{Corr\_std(n)}$$

. In the formula: p_corr, Corr_mean, Corr_std have the same meaning as represented by the variable names in steps SA1 and SA2.

. The matrix p_z (size of N x 1) is the final anomaly detection result.

. Option 2: Anomaly detection algorithm B, may specifically include the following steps SB1 to SB3.

. Step SB1, generating a baseline (a group brain connectivity matrix). It can specifically include the following sub-steps SB11 to SB14.

. Sub-step SB11, collecting data: collecting a large sample of fMRI data (generally, >=300 people).

. Sub-step SB12, pre-processing data: the data is pre-processed according to the pre-processing method in the embodiment of FIG. 2.

. Sub-step SB13, calculating a brain connectivity matrix.

. Here, the sub-steps SB11 to SB13 have the same content and effect as the sub-steps SA11 to SA13 in the step SA1 of the above described Option 1, and will not be repeated here.

. Sub-step SB14, generating a baseline.

. Calculating the mean matrix and the standard deviation matrix of indi_fc of all sample data, i.e., Big_mean_fc, Big_std_fc.

. Step S2, generating the FC of the subject (the brain connectivity matrix of the subject). Specifically, it may include the following sub-steps SB21 to SB22, wherein: sub-step SB21, calculating an average time sequence of all vertices/voxels in each ROI (generated by step 1) by using the pre-processed data of the subject (e.g., P).

. Sub-step SB22, sequentially calculating the correlation coefficients (e.g., using Pearson's correlation coefficient) between ROIs and ROI time sequences, and finally obtaining the brain connectivity matrix (functional connectivity, FC) of each subject, i.e., p_fc (the size of N x N).

. Sub-step SB3, anomaly detection algorithm step. Specifically, it may include the following.

. The following operations are performed on the data in $i^{th}$ row and $j^{th}$ column ($i \leq N$, $j \leq N$, N being the number of ROIs) of the p_fc matrix:

$$p\_z(i,j) = \frac{|p\_fc(i,j) - Big\_mean\_fc(i,j)|}{Big\_std\_fc(i,j)}$$

. where: p_fc, Big_mean_fc, Big_std_fc have the same meaning as represented by the variable name in the steps SB1 and SB2.

. Summing the values greater than the threshold k (k is an integer greater than or equal to 2) in each row of p_z (the size of N x 1), and finally obtaining the one-dimensional matrix of N x 1, i.e., p_z_sum (the size of N x 1), which is the final anomaly detection result.

. Here, the abnormality detection result is m (m>=0) brain regions obtained by the locating where the subject has an abnormality compared to the normal, i.e. m ROIs.

. In above step 203, the at least one abnormal ROI may be a brain region abnormal ROI or a cerebellum region abnormal ROI.

. Step 204, the target is determined based on the at least one abnormal ROI.

. In some optional embodiments, the above step 204 may specifically include:

. In some optional embodiments, determining the target based on the at least one abnormal ROI includes:

determining whether the at least one abnormal ROI is located in the modulation brain region or not;

if the at least one abnormal ROI is located in the modulation brain region, determining a center of the at least one abnormal ROI as the target, or, determining a region with the center of the at least one abnormal ROI as a spherical center and with a predetermined target radius as a first target ROI, determining the target based on a position of the first target ROI;

if the at least one abnormal ROI is not located in the modulation brain region, determining the connectivity of the at least one abnormal ROI with other ROIs in the at least two ROIs, and the ROI among the other ROIs having the connectivity with the at least one abnormal ROI that exceeds a predetermined connectivity threshold and which is located in the modulation region as a second target candidate;

determining a center of the second target candidate as the target, or determining a region with the center of the second target candidate as a spherical center and with the predetermined target radius as the second target ROI, determining the target based on a position of the second target ROI.

. In some optional embodiments, determining the target based on the at least one abnormal ROI includes:

determining a brain structure subdivision in which the target is located based on a disease type of the subject;

determining an intersection of the at least one abnormal ROI or the ROI whose connectivity with the abnormal ROI satisfies a predetermined connectivity threshold condition with the brain structure subdivision as a target candidate;

determining a center of the target candidate as the target, or, determining a region with the center of the target candidate as a spherical center and with a predetermined target radius as the target ROI, and determining the target based on a position of the target ROI.

. The disease type includes the type of disease determined by the diagnosis of the subject or the type of disease corresponding to the symptoms for which the subject is to be treated.

. The brain region correspondences of the disease types may be queried based on the existing brain region correspondences of the determined disease types, or they may be set according to actual needs. The method of obtaining the brain regions of the disease types herein is only an example, not a specific limitation.

. Here, the target brain region is the brain region corresponding to the target, and there is a neural correlation between the target and the target brain region, and the target brain region can be neuromodulated by stimulation to the target.

. The target may include coordinates corresponding to the single voxel, or it may be a collection of regions composed of a number of voxels.

. In some optional embodiments, the above step 204 may specifically comprise:
determining the central position of at least one target brain region as the target.

. In some optional embodiments, the above step 204 may specifically comprise:
determining a region with a center position of the at least one target brain region as a spherical center and a predetermined target radius as a region of interest(ROI) of the targets, identifying a position of the ROI of the targets as the target.

. The present disclosure does not specifically limit the length of the predetermined target radius, and the predetermined target radius may be set according to the actual needs of neuromodulation, for example, the predetermined target radius may be 3mm.

. In some optional embodiments, the above step 204 may specifically comprise:

> determining an intersection of the at least one target brain regions with the brain structural subdivisions, according to the brain structural subdivision in which the target is located;
> determining the targets in the intersection.

. In some optional embodiments, the target needs to fulfill the following conditions: the target may not be located in an inner surface and a base of the brain, the target may be located in the cerebral gyrus, and the target may not be located in the sulcus.

. The determination of the target is more accurate in accordance with the above method, and in practical application, the scientific researchers or medical personnel may conduct neuromodulation navigation on the subject using the optical navigation device or the electromagnetic navigation device according to the target determined in accordance with the above method, which can improve the efficiency of neuromodulation.

. The present disclosure provides a neuromodulation apparatus configured to neuromodulate the target of the subject in accordance with a predetermined neuromodulation solution, wherein the target of the subject is determined according to a method for target identification in any of the above embodiments of the present disclosure.

. The neuromodulation apparatus may include implantable neuromodulation apparatuses and non-implantable neuromodulation apparatuses, such as event-related potential analysis systems, electroencephalography systems, brain-computer interface devices, and the like. The present disclosure does not limit the specific forms of the neuromodulation apparatuses, which are only illustrated herein by way of example.

. The neuromodulation of the target of the subject may be modulated by the operator after connecting the neuromodulation apparatus in accordance with the target, or it may be modulated by the neuromodulation apparatus in accordance with the target of the subject obtained by input from the operator or obtained actively by the neuromodulation apparatus. This is only an example, not a specific limitation of neuromodulation of the target of the subject, and the technical persons may operate the neuromodulation apparatus according to the actual way of using the neuromodulation apparatus. Exemplarily, the predetermined neuromodulation solution may include, but is not limited to:

> a. Neuromodulation solution based on electrical pulse sequences
>
> > i. deep brain electrical stimulation
> > ii. transcranial electrical stimulation
> > iii. electroconvulsive related therapy
> > iv. electrical stimulation based on cortical brain electrodes
> > v. related derivative technologies of the above techniques
>
> b. Neuromodulation solution based on magnetic pulse sequences
>
> > i. transcranial magnetic stimulation and related solutions
> > ii. related derivative technologies of the above techniques
>
> c. Ultrasound-based neuromodulation solutions
>
> > i. focused ultrasound neuromodulation solution
> > ii. magnetic resonance-guided high-energy ultrasound focused therapy system and related modulation solutions

iii. derivative technologies of the above technologies

d. Light-based neuromodulation solutions

i. different wavelengths of light stimulation and related solutions
ii. derivative technologies of the above technologies

. With the gradual development of a new neuromodulation apparatus and neuromodulation technology, the method for target identification of the present disclosure may also be used to determine the target of neuromodulation in the future neuromodulation apparatus and neuromodulation solutions, which also fall within the scope of protection of the present disclosure.

. The embodiments of the present disclosure can efficiently and reliably obtain functional information of various parts of the brain by the method of establishing a precise individual brain map, which improves the accuracy of brain region location. Functional location with the aid of precise individual-level brain map improves the reliability of the results of neuromodulation target location.

. Referring further to FIG. 5, as an implementation of the method shown in each of the above figures, the present disclosure provides an embodiment of a device for target identification, the device embodiment of which corresponds to the method embodiment shown in FIG. 2, and which can be specifically applied in various electronic apparatus.

. As shown in FIG. 5, the device for target identification 500 of the present embodiment includes: an data acquisition unit 501, a processing unit 502, an anomaly detection unit 503, and a target identification unit 504.

. The data acquisition unit 501 is configured to acquire the scanning data of the subject, wherein the scanning data comprise data acquired from magnetic resonance imaging of the brain of the subject, the scanning data comprising a blood oxygen level dependency BOLD signal sequence corresponding to each voxel in a predetermined number of voxels; the processing unit 502 is configured to determine, on the basis of the scanning data, at least two ROIs of the subject; the anomaly detection unit 503 is configured to determine, based on the predetermined anomaly detection rule, at least one abnormal ROI in the at least two ROIs; and the target identification unit 504 is configured to determine the target based on the at least one abnormal ROI.

. In some optional embodiments, the processing unit 502 is further configured to:

obtain the BOLD signal sequence corresponding to each voxel in the scanning data;
determine, based on the BOLD signal sequence corresponding to each voxel, the connectivity between each two voxels in the scanning data, forming the brain connectivity matrix of the subject corresponding to the scanning data;
forming at least two ROIs based on the brain region template of the standard brain and the brain connectivity matrix of the subject.

. In some optional embodiments, the processing unit 502 is further configured to:

obtain the BOLD signal sequence corresponding to each voxel in the scanning data
determine, based on the BOLD signal sequence corresponding to each voxel, the connectivity between each two voxels in the scanning data;
divide the scanning data corresponding to the anatomical structure of the brain of the subject into a plurality of big regions, dissect each of the plurality of big regions into a plurality of brain regions, wherein each of the plurality of brain regions includes at least one voxel;
fuse brain regions of the plurality of brain regions having a voxel connectivity between the brain regions above a predetermined brain region voxel connectivity threshold to form the at least two ROIs.

. In some optional embodiments, the processing unit 502 is further configured to:
determine the at least two ROIs of the subject based on a volume standard brain template according to the scanning data.
. In some optional implementations, the processing unit 502 is further configured to:
determine the at least two ROIs of the subject based on a cortical standard brain template according to the scanning data.
. In some optional embodiments, the anomaly detection unit 503 is further configured to:

acquire group brain magnetic resonance data;
determine a group brain connectivity matrix based on the group brain magnetic resonance data;
acquire the BLOD signal sequence corresponding to each voxel in the scanning data;
determine connectivity between each two voxels in the scanning data based on the BLOD signal sequence, to form a brain connectivity matrix of the subject corresponding to the scanning data;
determine the at least one abnormal ROI in accordance with the group brain connectivity matrix and the brain connectivity matrix of the subject

. In some optional embodiments, the target identification unit 504, is further configured to: determine the target based on the at least one abnormal ROI, comprising:

determine whether the at least one abnormal ROI is located in a modulation brain region or not;
if the at least one abnormal ROI is located in the modulation brain region, determining a center of the at least one abnormal ROI as the target, or, determining a region with the center of the at least one abnormal ROI as a spherical center and with a predetermined target radius as a first target ROI, determining the target based on a position of the first target ROI;
if the at least one abnormal ROI is not located in the modulation brain region, determining the connectivity of the at least one abnormal ROI with other ROIs in the at least two ROIs, and the ROI among the other ROIs having the connectivity with the at least one abnormal ROI that exceeds a predetermined connectivity threshold and which is located in the modulation region as a second target candidate;
determining a center of the second target candidate as the target, or determining a region with the center of the second target candidate as a spherical center and with the predetermined target radius as the second target ROI, determining the target based on a position of the second target ROI.

. In some optional embodiments, determining the target based on the at least one abnormal ROI includes:

determining a brain structure subdivision in which the target is located based on a disease type of the subject;
determining an intersection of the at least one abnormal ROI or the ROI whose connectivity with the abnormal ROI satisfies a predetermined connectivity threshold condition with the brain structure subdivision as a target candidate;
determining a center of the target candidate as the target, or, determining a region with the center of the target candidate as a spherical center and with a predetermined target radius as the target ROI, and determining the target based on a position of the target ROI.

. In some optional embodiments, the functional magnetic resonance imaging includes: structural magnetic resonance imaging, and/or, task-based functional magnetic resonance imaging, and/or, resting state functional magnetic resonance imaging.

. It is noted that the implementation details and technical effects of the units in the device for target identification provided in the present disclosure can be referred to other embodiments of the present disclosure and will not be repeated herein.

. Referring now to FIG. 6, it shows a structural schematic diagram of a computer system 600 suitable for use in implementing a terminal device or server of the present disclosure. The terminal device or server shown in FIG. 6 is only an example, and should not make any limitation to the function and the use range of the present disclosure.

. As shown in FIG. 6, the computer system 600 includes a Central Processing Unit (CPU) 601 which can perform various appropriate actions and processes according to a program stored in a Read Only Memory (ROM) 602 or a program loaded from a storage section 608 into a Random Access Memory (RAM) 603. In the RAM 603, various programs and data necessary for the operation of the computer system 600 are also stored. The CPU 601, ROM 602, and RAM 603 are connected to each other via a bus 604. An Input/Output (I/O) interface 605 is also connected to the bus 604.

. The following components are connected to the I/O interface 605: an input section 606 including a keyboard, a mouse, and the like; an output section 607 including such as a Cathode Ray Tube (CRT), a Liquid Crystal Display (LCD), and a speaker; a storage section 608 including a hard disk and the like; and a communication section 609 including a network interface card such as a LAN (Local Area Network) card, a modem. The communication section 609 performs communication processing via a network such as the Internet.

. In particular, the processes described above with reference to the flow charts may be implemented as computer software programs, according to embodiments of the present disclosure. For example, embodiments of the present disclosure include a computer program product including a computer program carried on a computer-readable medium, the computer program including program code for performing the method illustrated by the flow chart. In such an embodiment, the computer program can be downloaded and installed from the network via communication section 609. The computer program performs the above-described functions defined in the method of the present disclosure when executed by the Central Processing Unit (CPU) 601. It should be noted that the computer readable medium of the present disclosure can be a computer readable signal medium or a computer readable storage medium or any combination of both. A computer readable storage medium may be, for example, but not limited to, an electric, magnetic, optical, electromagnetic, infrared, or semiconductor system, apparatus, or device, or any combination of thereof. More specific examples of the computer readable storage medium may include, but are not limited to: an electrical connection having at least one wires, a portable computer disk, a hard disk, a Random Access Memory (RAM), a read-only memory (ROM), an erasable programmable read-only memory (EPROM or flash memory), an optical fiber, a portable compact disc read-only memory (CD-ROM), an optical storage device, a magnetic storage device, or any suitable combination of thereof. In the present disclosure, the computer readable storage medium may be any tangible medium that can contain, or store a program for use by or in connection with an instruction execution system, apparatus, or device. In contrast, in the present disclosure, a computer-readable signal medium may include a propagated data signal carried with computer-readable program code therein in base band or as part of a carrier wave. Such a propagated data signal may take a variety of forms, including, but not limited to, electromagnetic signals, optical signals, or any suitable combination thereof. The computer readable signal medium may be any computer readable medium that is other than a computer readable storage medium and that can communicate, propagate, or transmit a program for use by or in connection with an instruction execution system, apparatus, or device. Program code embodied on a computer readable medium may be transmitted using any appropriate medium, including but not limited to: wireless, wire, fiber optic cable, RF, etc., or any suitable combination of thereof.

. Computer program code for carrying out operations for the present disclosure may be written in one or more programming languages or any combination thereof, including object oriented programming languages such as Java, Smalltalk, C++, Python, and conventional procedural programming languages, such as "C" programming language or similar programming languages. The program code may execute entirely on the user's computer, partly on the user's computer, as a standalone software package, partly on the user's computer and partly on a remote computer or entirely on the remote computer or server. In the scenario associated with the remote computer, the remote computer may be connected to the user's computer through any type of networks, including a Local Area Network (LAN) or a Wide Area Network (WAN), or the connection may be made to an external computer (for example, through the Internet using an Internet service provider).

. The flowchart and block diagrams in the figures illustrate the architecture, functionality, and operation of possible implementations of systems, methods and computer program products according to various embodiments of the present disclosure. In this regard, each block in the flowchart or block diagrams may represent a module, program segment, or a portion of code, which includes at least one executable instructions for implementing the specified logical function(s). It should also be noted that, in some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart, and combinations of blocks in the block diagrams and/or flowchart, can be implemented by special purpose hardware-based systems that perform the specified functions or acts, or combinations of special purpose hardware and computer instructions.

. The units described in the present disclosure may be implemented by software or hardware. The described units may also be provided in a processor, which may be described as: a processor includes a scanning data acquisition unit, a setting unit, a processing unit, and a target identification unit. The names of the units do not constitute a limitation on the units themselves in some way.

. As another aspect, the present disclosure also provides a computer-readable medium, which may be contained in the device described in the above embodiments; or may be separate and not assembled into the device. The computer readable medium carries at least one program which, when executed by the device, causes the device to: acquiring scanning data of a subject, wherein the scanning data comprise the data acquired from magnetic resonance imaging of the brain of the subject, the scanning data comprise a BOLD signal sequence corresponding to each voxel of a preset number of voxels; determine at least two regions of interest (ROI) of the subject based on the scanning data; determine at least one abnormal region of interest in the at least two regions of interest in accordance with a predetermined anomaly detection rule; determine a target based on the at least one abnormal region of interest.

. The foregoing description is only exemplary of the preferred embodiments of the present disclosure and is illustrative of the principles of the technology employed. It will be appreciated by those skilled in the art that the scope of the invention in the present disclosure is not limited to the technical solutions defined by the specific combination of the above-mentioned technical features, but also encompasses other technical solutions in which any combination of the above-mentioned features or their equivalents is made without departing from the inventive spirit of the application. For example, such other technical solutions may be defined by the above features and the technical features disclosed in the present disclosure (but not limited to) having similar functions are replaced with each other.

. The technical solutions described in the embodiments of the present disclosure can be arbitrarily combined without conflict.

. The above description is only for the specific embodiments of the present disclosure, but the protection scope of the present disclosure is not limited thereto, and any person skilled in the art can easily think of the changes or substitutions within the technical scope of the present disclosure, and shall cover the protection scope of the present disclosure. Therefore, the protection scope of the present disclosure shall be defined by the protection scope of the appended claims.

**Claims**

1. A computer-implemented method for target identification of a subject to realize the location of individualized neuromodulation target of the subject, comprising:

   Acquiring (201) scanning data of the subject, wherein the scanning data comprise the data acquired from magnetic resonance imaging of the brain of the subject;
   Determining (202) at least two regions of interest of the subject based on the scanning data; **characterized in that**,
   the computer-implemented method further comprises:

   Determining (203) at least one abnormal region of interest in the at least two regions of interest in accordance with a predetermined anomaly detection rule; and
   Determining (204) a target based on the at least one abnormal region of interest,
   wherein determining a target based on the at least one abnormal region of interest comprises:

   determining whether the at least one abnormal region of interest is located in a modulation brain region or not;
   if the at least one abnormal region of interest is located in the modulation brain region, determining a center of the at least one abnormal region of interest as the target, or, determining a region with the center of the at least one abnormal region of interest as a spherical center and with a predetermined target radius as a first target region of interest, determining the target based on a position of the first target region of interest;
   if the at least one abnormal region of interest is not located in the modulation brain region, determining the connectivity of the at least one abnormal region of interest with other regions of interest in the at least two regions of interest, and the region of interest among the other regions of interest having the connectivity with the at least one abnormal region of interest that exceeds a predetermined connectivity threshold and which is located in the modulation region as a second target candidate; and
   determining a center of the second target candidate as the target, or determining a region with the center of the second target candidate as a spherical center and with the predetermined target radius as the second target region of interest, determining the target based on a position of the second target region of interest.

2. The computer-implemented method according to claim 1, wherein determining the at least two regions of interest of the subject based on the scanning data, comprises:
   determining the at least two regions of interest of the subject based on a volume standard brain template according to the scanning data.

3. The computer-implemented method according to claim 1, wherein determining the at least two regions of interest of the subject based on the scanning data, comprises:
   determining the at least two regions of interest of the subject based on a cortical standard brain template according to the scanning data.

4. The computer-implemented method according to claim 1, wherein determining the at least two regions of interest of the subject based on the scanning data, comprises:

   Determining (202a1) connectivity between each two voxels in the scanning data to form a brain connectivity matrix corresponding to the scanning data; and
   Forming (202a2) the at least two regions of interest based on a brain region template of a standard brain and the brain connectivity matrix.

5. The computer-implemented method according to claim 1, wherein determining at least one abnormal region of interest in the at least two regions of interest in accordance with a predetermined anomaly detection rule, comprises:

   acquiring group brain magnetic resonance data;
   determining a group brain connectivity matrix based on the group brain magnetic resonance data;
   determining connectivity between each two voxels in the scanning data to form a brain connectivity matrix of the subject corresponding to the scanning data; and

determining the at least one abnormal region of interest in accordance with the group brain connectivity matrix and the brain connectivity matrix of the subject.

6. The computer-implemented method according to claim 1, wherein determining the target based on the at least one abnormal region of interest comprises:

   determining a brain structure subdivision in which the target is located based on a disease type of the subject; determining an intersection of the at least one abnormal region of interest or the region of interest whose connectivity with the abnormal region of interest satisfies a predetermined connectivity threshold condition with the brain structure subdivision as a target candidate; and
   determining a center of the target candidate as the target, or, determining a region with the center of the target candidate as a spherical center and with a predetermined target radius as the target region of interest, and determining the target based on a position of the target region of interest.

7. The computer-implemented method according to claim 1, wherein the magnetic resonance imaging comprises: structural magnetic resonance imaging of the brain, and/or, task-based functional magnetic resonance imaging, and/or, resting state functional magnetic resonance imaging.

8. An electronic apparatus, comprising:

   at least one processor; and
   a storage device having at least one program stored thereon,
   wherein the at least one program, when executed by the at least one processor, causes the at least one processor to execute the computer-implemented method according to any one of claims 1 to 7.

9. A computer readable storage medium, having a computer program stored thereon, wherein the computer program, when executed by at least one processor, executes the method according to any one of claims 1 to 7.

10. A neuromodulation apparatus comprising the electronic apparatus of claim 8 , configured to make neuromodulation on a target of a subject in accordance with a preset neuromodulation solution; wherein the target is determined by the computer-implemented method according to any one of claims 1 to 7.

11. The apparatus according to claim 10, wherein the preset neuromodulation solution comprises at least one of:

   deep brain electrical stimulation;
   transcranial electrical stimulation;
   electroconvulsive therapy;
   electrical stimulation based on cortical brain electrodes;
   transcranial magnetic stimulation;
   focused ultrasound neuromodulation;
   magnetic resonance guided high-intensity focused ultrasound therapy neuromodulation; and
   photobiomodulation therapy.


**Patentansprüche**

1. Computergestütztes Verfahren zur Zielpunktidentifizierung eines Probanden zur Lokalisierung eines individualisierten Neuromodulationszielpunkts, umfassend:

   Erfassen (201) von Scandaten des Probanden, wobei die Scandaten die mittels Magnetresonanztomographie des Gehirns des Probanden erfassten Daten umfassen;
   Bestimmen (202) von mindestens zwei Regionen von Interesse des Probanden basierend auf den Scandaten;
   **dadurch gekennzeichnet, dass**
   computergestütztes Verfahren ferner Folgendes umfasst:

   Bestimmen (203) von mindestens einer abnormalen Region von Interesse in den mindestens zwei Regionen von Interesse gemäß einer vorbestimmten Anomalieerkennungsregel; und
   Bestimmen (204) eines Zielpunkts basierend auf der mindestens einen abnormalen Region von Interesse,

wobei das Bestimmen eines Zielpunkts basierend auf der mindestens einen abnormalen Region von Interesse Folgendes umfasst:

Bestimmen, ob sich die mindestens eine abnormale Region von Interesse in einer modulierbaren Hirnregion befindet oder nicht;
Befindet sich die mindestens eine abnormale Region von Interesse in der Modulationsregion des Gehirns, der Mittelpunkt dieser mindestens einen abnormalen Region von Interesse als Zielpunkt bestimmt wird; oder eine Region mit dem Mittelpunkt dieser mindestens einen abnormalen Region von Interesse als Kugelmittelpunkt und ein vorgegebener Zielpunktradius als erste Zielpunktregion von Interesse bestimmt wird, der Zielpunkt basierend auf einer Position dieser ersten Zielpunktregion von Interesse bestimmt wird,
Befindet sich die mindestens eine abnormale Region von Interesse nicht in der Modulationsregion des Gehirns, die Konnektivität dieser Region mit anderen Regionen von Interesse in den mindestens zwei Regionen von Interesse bestimmt wird, diejenige Region von Interesse, deren Konnektivität mit der mindestens einen abnormalen Region von Interesse einen vorgegebenen Schwellenwert überschreitet und die sich in der Modulationsregion befindet, als zweite Zielpunktkandidaten ausgewählt wird, der Mittelpunkt dieser zweiten Zielpunktkandidaten von Interesse als Zielpunkt bestimmt wird, oder eine Region mit dem Mittelpunkt dieser zweiten Zielpunktkandidaten als Kugelmittelpunkt und ein vorgegebener Zielpunktradius als zweite Zielpunktregion von Interesse bestimmt wird, der Zielpunkt basierend auf einer Position dieser zweiten Zielpunktregion von Interesse bestimmt wird.

2. Computergestütztes Verfahren nach Anspruch 1, wobei die Bestimmung der mindestens zwei Regionen von Interesse des Probanden basierend auf den Scandaten Folgendes umfasst:
Bestimmung der mindestens zwei Regionen von Interesse des Probanden basierend auf einer volumetrischen Standard-Gehirnvorlage gemäß den Scandaten.

3. Computergestütztes Verfahren nach Anspruch 1, wobei die Bestimmung der mindestens zwei Regionen von Interesse des Probanden basierend auf den Scandaten Folgendes umfasst:
Bestimmung der mindestens zwei Regionen von Interesse des Probanden basierend auf einer kortikalen Standard-Gehirnvorlage gemäß den Scandaten.

4. Computergestütztes Verfahren nach Anspruch 1, wobei die Bestimmung der mindestens zwei Regionen von Interesse des Probanden basierend auf den Scandaten Folgendes umfasst:

Bestimmen (202a1) der Konnektivität zwischen je zwei Voxeln in den Scandaten zur Bildung einer den Scandaten entsprechenden Gehirnkonnektivitätsmatrix; und
Bilden (202a2) der mindestens zwei Regionen von Interesse basierend auf einer Gehirnregionsvorlage eines Standardgehirns und der Gehirnkonnektivitätsmatrix.

5. Computergestütztes Verfahren nach Anspruch 1, wobei die Bestimmung mindestens einer abnormalen Region von Interesse in den mindestens zwei Regionen von Interesse gemäß einer vorgegebenen Anomalieerkennungsregel Folgendes umfasst:

Erfassen von Gruppen-Magnetresonanzdaten des Gehirns;
Bestimmen einer Gruppen-Konnektivitätsmatrix des Gehirns basierend auf den Gruppen-Magnetresonanzdaten des Gehirns;
Bestimmen der Konnektivität zwischen je zwei Voxeln in den Scandaten zur Bildung einer den Scandaten entsprechenden Gehirnkonnektivitätsmatrix des Probanden; und
Bestimmen der mindestens einen abnormalen Region von Interesse gemäß der Gruppen-Konnektivitätsmatrix des Gehirns und der Gehirnkonnektivitätsmatrix des Probanden.

6. Computergestütztes Verfahren nach Anspruch 1, wobei die Bestimmung des Zielpunkts basierend auf der mindestens einen abnormalen Region von Interesse Folgendes umfasst:

Bestimmen einer Unterteilung der Gehirnstruktur, in der sich der Zielpunkt basierend auf dem Krankheitstyp des Probanden befindet;
Bestimmen des Schnittpunkts der mindestens einen abnormalen Region von Interesse oder der Region von Interesse, deren Konnektivität mit der abnormalen Region von Interesse eine vorgegebene Konnektivitätsschwellenbedingung erfüllt, wobei die Gehirnstrukturunterteilung als Zielpunktkandidaten ausgewählt wird, und Bestimmen des Mittelpunkts des Zielpunktkandidaten als Zielpunkt oder Bestimmen einer Region mit dem Mittelpunkt des Zielpunktkandidaten als Kugelmittelpunkt, wobei ein vorgegebener Zielpunktradius als Zielpunktregion bestimmt wird, und der Zielpunkts basierend auf einer Position der Zielpunktregion von Interesse bestimmt wird.

7. Computergestütztes Verfahren nach Anspruch 1, wobei die Magnetresonanztomographie Folgendes umfasst: strukturelle Magnetresonanztomographie des Gehirns und/oder aufgabenbasierte funktionelle Magnetresonanztomographie und/oder funktionelle Magnetresonanztomographie im Ruhezustand.

8. Elektronische Vorrichtung, umfassend:

   mindestens einen Prozessor; und
   ein Speichermedium mit mindestens einem darauf gespeicherten Programm,
   wobei das mindestens eine Programm, wenn es vom mindestens einen Prozessor ausgeführt wird, diesen Prozessor veranlasst, computerimplementiertes Verfahren gemäß einem der Ansprüche 1 bis 7 auszuführen.

9. Computerlesbares Speichermedium, mit einem darauf gespeicherten Computerprogramm, wobei das Computerprogramm, wenn es von mindestens einem Prozessor ausgeführt wird, das Verfahren gemäß einem der Ansprüche 1 bis 7 ausführt.

10. Neuromodulationsvorrichtung, umfassend die elektronische Vorrichtung nach Anspruch 8, diese Neuromodulationsvorrichtung so konfiguriert ist, dass sie eine Neuromodulation an einer Zielpunktregion eines Probanden gemäß einer voreingestellten Neuromodulationslösung durchführt; wobei der Zielpunkt mittels des computergestützten Verfahrens nach einem der Ansprüche 1 bis 7 bestimmt wird.

11. Vorrichtung nach Anspruch 10, wobei die voreingestellte Neuromodulationslösung mindestens eine der folgenden Methoden umfasst:

   tiefe elektrische Hirnstimulation;
   transkranielle elektrische Stimulation;
   Elektrokrampftherapie;
   elektrische Stimulation basierend auf kortikalen Hirnelektroden;
   transkranielle Magnetstimulation;
   fokussierte Ultraschall-Neuromodulation;
   Magnetresonanz-gesteuerte hochintensive fokussierte Ultraschalltherapie-Neuromodulation; und
   Photobiomodulationstherapie.

**Revendications**

1. Procédé mis en œuvre par ordinateur pour l'identification d'une cible chez un sujet, destiné à déterminer l'emplacement d'une cible de neuromodulation individualisée chez ledit sujet, comprenant:

   l'acquisition (201) de données d'imagerie du sujet, lesdites données d'imagerie comprenant les données acquises à partir d'une imagerie par résonance magnétique du cerveau du sujet ;
   la détermination (202) d'au moins deux régions d'intérêt du sujet sur la base des données d'imagerie ;
   **caractérisé en ce que**,
   le procédé mis en œuvre par ordinateur comprend en outre :

      la détermination (203) d'au moins une région d'intérêt anormale parmi les au moins deux régions d'intérêt, conformé ment à unerègle de détection d'anomalie prédéterminée ; et
      Déterminer (204) une cible sur la base de la au moins une région d'intérêt anormale,
      dans lequel la détermination d'une cible sur la base de la au moins une région d'intérêt anormale comprend:

         déterminer si la au moins une région d'intérêt anormale est situé e ou non dans une région cérébrale de modulation;
         si la au moins une région d'intérê t anormale est situé e dans la région cérébrale de modulation, déterminer un centre de la au moins une région d'intérêt anormale comme cible, ou, déterminer une région ayant pour centre le centre de la au moins une région d'intérê t anormale et un rayon cible prédéterminé comme premièr région d'intérêt cible, déterminer la cible sur la base d'une position de la première région d'intérêt cible;

si la au moins une région d'intérêt anormale n'est pas située dans la région cérébrale de modulation, déterminer la connectivité de la au moins une région d'intérêt anormale avec d'autres régions d'intérêt parmi les au moins deux régions d'intérêt, et la région d'intérêt parmi les autres régions d'intérêt ayant une connectivité avec la au moins une région d'intérêt anormale qui dépasse un seuil de connectivité prédéterminé et qui est situé e dans la région de modulation comme deuxième candidate cible ; et

déterminer un centre de la deuxiè me cible candidate comme cible, ou déterminer une région ayant pour centre le centre de la deuxième cible candidate et pour rayon la valeur prédé terminé e comme deuxième région d'intér t cible, déterminer la cible en fonction d'une position de la deuxième région d'intérêt cible.

2. Procédé mis en œuvre par ordinateur selon la revendication 1, dans lequel la détermination des au moins deux zones d'intérêt du sujet sur la base des données d'imagerie comprend :
la détermination des au moins deux zones d'intérêt du sujet sur la base d'un modèle cérébral standard en volume, conformé ment aux donné es d'imagerie.

3. Procédé mis en œuvre par ordinateur selon la revendication 1, dans lequel la détermination des au moins deux régions d'intérêt du sujet sur la base des données de balayage comprend :
la détermination des au moins deux régions d'intérêt du sujet sur la base d'un modèle cérébral cortical standard en fonction des donné es de balayage.

4. Procédé mis en œuvre par ordinateur selon la revendication 1, dans lequel la détermination des au moins deux régions d'intérêt du sujet sur la base des données d'imagerie comprend :

la détermination (202a1) de la connectivité entre chaque paire de voxels dans les données d'imagerie afin de former une matrice de connectivité cérébrale correspondant aux données d'imagerie ; et
la formation (202a2) des au moins deux régions d'intérêt sur la base d'un modèle de région cérébrale d'un cerveau standard et de la matrice de connectivité cérébrale.

5. Procédé mis en œuvre par ordinateur selon la revendication 1, dans lequel la détermination d'au moins une région d'intérêt anormale parmi les au moins deux r é gions d'int rêt conformé ment à une règle dedétection d'anomalie prédéterminée comprend :

l'acquisition de donné es de r é sonance magnétique cérébrale de groupe ;
déterminer une matrice de connectivité cérébrale de groupe sur la base des données de résonance magnétique cérébrale de groupe ;
déterminer la connectivité entre chaque paire de voxels dans les données d'imagerie afin de former une matrice de connectivité cérébrale du sujet correspondant aux données d'imagerie ; et
dé terminer la au moins une région d'intérêt anormale conformément à la matrice de connectivité cérébrale de groupe et à la matrice de connectivité cérébrale du sujet.

6. Proc é dé mis en œuvre par ordinateur selon la revendication 1, dans lequel la détermination de la cible sur la base de la au moins une région d'intérêt anormale comprend :

la détermination d'une subdivision de la structure cérébrale dans laquelle la cible est située sur la base d'un type de maladie du sujet ;
la détermination d'une intersection de la au moins une région d'intérêt anormale ou de la région d'intérêt dont la connectivité avec la région d'intérêt anormale satisfait à une condition de seuil de connectivité prédéterminée avec la subdivision de la structure cérébrale en tant que candidate cible ; et
la détermination d'un centre de la cible candidate en tant que cible, ou la détermination d'une région ayant pour centre le centre de la cible candidate et un rayon prédéterminé en tant que région d'intérêt cible, et la détermination de la cible sur la base d'une position de la région d'intérêt cible.

7. Procédé mis en œuvre par ordinateur selon la revendication 1, dans lequel l'imagerie par r ésonance magnétique comprend : une imagerie par résonance magnétique structurelle du cerveau, et/ou une imagerie par résonance magnétique fonctionnelle basée sur une tâche, et/ou une imagerie par résonance magnétique fonctionnelle à l'état de repos.

8. Appareil électronique, comprenant :

au moins un processeur ; et
un dispositif de stockage sur lequel est stock é au moins un programme,
dans lequel ledit au moins un programme, lorsqu' il est exécuté par ledit au moins un processeur, amène ledit au moins un processeur à exécuter le procédé mis en œuvre par ordinateur selon l' une quelconque des revendications 1 à 7.

9. Support de stockage lisible par ordinateur, sur lequel est stock é un programme informatique, dans lequel ledit programme informatique, lorsqu' il est exécuté par au moins un processeur, exécute le procédé selon l' une quelconque des revendications 1 à 7.

10. Appareil de neuromodulation, comprenant l'appareil électronique de la revendication 8, configuré pour effectuer une neuromodulation sur une cible d'un sujet conformé ment à une solution de neuromodulation prédéfinie ; dans lequel la cible est déterminé e par le procédé mis en œuvre par ordinateur selon l'une quelconque des revendications 1 à 7.

11. Appareil selon la revendication 10, dans lequel la solution de neuromodulation prédéfinie comprend au moins l'un des éléments suivants :

une stimulation électrique profonde du cerveau ;
une stimulation électrique transcrânienne ;
une thé rapie é lectroconvulsive ;
une stimulation électrique bas é e sur des électrodes corticales ;
une stimulation magnétique transcrânienne ;
une neuromodulation par ultrasons focalisés ;
une neuromodulation par thé rapie par ultrasons focalisé s de haute intensité guidé e par r é sonance magnétique ; et
une thé rapie par photobiomodulation.

_100_

## FIG. 1

_200_

201
acquiring scanning data of a subject

202
determining at least two ROIs of the subject based on the scanning data

203
determining at least one anomalous ROI in the at least two ROIs in accordance with a predetermined anomaly detection rule;

204
determining a target based on the at least one anomalous ROI

## FIG. 2

202

202a1

determining connectivity between each two voxels in the scanning data to form a brain connectivity matrix corresponding to the scanning data

202a2

forming the at least two ROIs based on a brain region template of a standard brain and the brain connectivity matrix

**FIG. 3**

202

202b1

determining the connectivity between each two voxels in the scanning data

202b2

dividing the scanning data corresponding to the anatomical structure of the brain of the subject into a plurality of big regions, dissecting the plurality of big regions into a plurality of brain regions, wherein each of the plurality of brain regions comprises at least one voxel

202b3

fusing brain regions of the plurality of brain regions having a voxel connectivity between the brain regions above a predetermined brain region voxel connectivity threshold to form the at least two ROIs

**FIG. 4**

500

501

data acquisition unit

502

processing unit

503

anomaly detection unit

504

target identification unit

**FIG. 5**

600

601

CPU

602

ROM

603

RAM

604

605

I/O interface

Input section

606

Output section

607

Storage section

608

Communication section

609

**FIG. 6**

**EP 4 342 373 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015119689 A1
- US 2021170180 A1